Europäisches Patentamt

European Patent Office

Office européen des brevets

(11) Publication number: **0 369 294**
**A2**

# (12) EUROPEAN PATENT APPLICATION

(21) Application number: **89120610.4**

(22) Date of filing: **07.11.89**

(51) Int. Cl.⁵: **B60J 3/00, A61L 9/12**

(30) Priority: **07.11.88 ES 8803274 U**

(43) Date of publication of application:
**23.05.90 Bulletin 90/21**

(84) Designated Contracting States:
**BE DE FR GB IT SE**

(71) Applicant: **INDUSTRIAS TECHNO-MATIC, S.A.**
**Calle Espronceda 324**
**E-08027 Barcelona(ES)**

(72) Inventor: **Cebollero, Gabas Carlos**
**180, Calle Rocafort**
**E-08029 Barcelona(ES)**

(74) Representative: **Dipl.-Ing. Schwabe, Dr. Dr.**
**Sandmair, Dr. Marx**
**Stuntzstrasse 16**
**D-8000 München 80(DE)**

(54) **Air freshener box for an automobile.**

(57) An air freshener box for an automobile is essentially formed by a main, hollow container or body incorporated in the structure of the sun visor of an automobile; said container is generally aligned with one of the main axes of said sun visor and is provided with a sliding cover mounted in respective lateral guideways disposed close to an upper edge and extending by means of corresponding extensions sufficiently beyond the edge of the container to allow for a major portion of the length of the cover to be received when said cover is moved into the opening position.

Fig.1

EP 0 369 294 A2

The invention relates to an air freshener box for an auto-mobile.

The employment of aromatic chemicals in automobiles to neutralise or mask both the characteristic smells of new vehicles and the smells generated by normal use is well known. The means proposed hithertofore for this purpose comprise basically solid or liquid aromatic substances employed in many shapes and on many carriers; such carriers are mounted inside the vehicle, attached either by adhesives to the bodywork or by simply suspending them in more or less accessible or totally inadequate places as far as the occupants' security is concerned. Furthermore there are many possibilities known to incorporate more or less complex ancillary, complementary items which aid driving or make the stay inside the vehicle more comfortable and pleasant.

It is the object of the invention to propose an air freshener box without the above disadvantages. A box for housing an air freshener element with a solid support is especially to be suggested, which forms a part of the structure of the sun visors with which automobiles are usually equipped.

In order to present a solution to the problem raised, i.e. the advantageous freshening of the interior of automobiles, a special device is described consisting of a box for containing air-freshening products specifically designed for incor poration as original equipment into the sun visor structures of automobiles.

Said air freshener box according to the invention provides the following basic advantages:

A) It may be adapted to any sun visor structure, irrespective of the type and size of the vehicle in question;

B) It has a design allowing any appropriate air freshener element to be housed, fixed, concealed and replaced with extreme ease;

C) The air freshener element is concealed by a cover designed for this purpose which slides on guideways provided in the main body of the box; and

D) The cover mentioned in above item C) is provided with orifices allowing the aroma of the air freshener product to issue; this effect is enhanced by the fact that the sun visor of an automobile is generally located in an area where the air flow from the front inner ventilation means of an automobile converges, so that said air flow entrains said aroma und thus provides for its extensive and speedy diffusion throughout the whole passanger compartment of the vehicle.

It is characteristic of the air freshener box according to the invention that it is essentially formed by a main hollow container or body having an essentially parallelepipedic or other appropriate shape incorporated into the structure of the auto-mobile sun visor. This container is generally aligned with one of the main axes of said sun visor and provided with a sliding cover mounted on respective lateral guideways located close to the upper edge of the container. These guideways extend sufficiently beyond the container's edge to allow the accomodation of a major portion of the cover's length when it is moved to open said container.

The air freshener box according to the invention comprises also structural reinforcement means which ensure and maintain an appropriate solidity and rigidity of the portions of the lateral guideways which extend beyond the perimeter of the main body and on which the air freshener box cover slides.

It is understood that specific means may be selected depending on the particular form to be manufactured, such as cross-members, gussets, plates and other elements of different shapes.

It is a further feature of the air freshener box according to the invention that it is provided with a plurality of outwardly projecting members which may be located in different positions to hold or fix the air freshener element.

The container is designed to be capable of accepting a large variety of differently shaped air freshener elements. With this end in mind, the attachment means are so designed that they accept all kinds of air freshener elements, but there is also the possibility of providing, in certain cases, attachment or holding means specifically devised for particular types of air freshener elements.

The air freshener box according to the invention is also characterised in that its cover is provided with aeration orifices facilitating the diffusion of the air freshener aroma throughout the interior of the vehicle. Furthermore the user can move the cover, for example, by means of a projection which may be easily manipulated with the fingers.

In the sheet of drawings accompanying the present specification, the air freshener box for the sun visor of an automobile is shown as follows:

Figure 1 is a plan view of an embodiment of such a box,

Figure 2 is a plan view of the same embodiment in a different operative position, and

Figure 3 is a cross section view along the line III-III of Figure 1.

The air freshener box of the invention for an automobile sun visor as embodied in the accompanying drawings is formed essentially by a main body or container 1 and a cover 2 thereof, both of which are made from material appropriate for its designated use and purpose, for example plastic, and can be adapted to the sun visor structure 3.

Said container 1 with its cover 2 can be incorporated as original equipment, i.e. by the manufacturer, in the sun visor structure 3 and they thus have a correspondingly adapted shape, size and configuration.

The container 1 has in the embodiment shown an essentially parallelepipedic shape and is aligned with the main axis of the sun visor 3. The container 1 houses an air freshener element 4. Both the shape and the surface defining the access to the interior of the container 1 are formed to facilitate the exchange and use of the air freshener element 4 necessary under regular conditions of use.

The cover 2 is moveable, so that it may slide easily during the opening and closing of the container 1. For this purpose lateral guideways 5 and 5' are provided close to the upper edge 6 of the container 1. When the container 1 is provided with straight, parallel edges, as is the case in the embodiment illustrated in the drawings, the guideways or rails 5-5' may be disposed on the same inner wall of the container itself.

When, however, the container is oval or circular, corresponding rail-like projections mounting the guideways 5-5' are provided.

Furthermore, in all cases (parallelepipedic, oval or circular container 1), the guideways 5-5' are continued as corresponding extensions 5p-5p' beyond the container 1 so that almost the whole length of the cover 2 can be accomodated when the container 1 is opened. These extensions 5p-5p' of the guideways 5-5' generally require reinforcements to ensure their positional stability and strength.

In the embodiment shown, rails 7-7' and the cross wall 8 are used as reinforcements. Said rails 7-7' have such a length that the cover 2 can move beyond the container 1 when opened, thereby defining a sliding plane for the cover 2 essentially parallel to the base plane of the box of the invention. Both longitudinal and transverse ribs may be provided with appropriate dimensions to ensure that the box has the desired strength and stability.

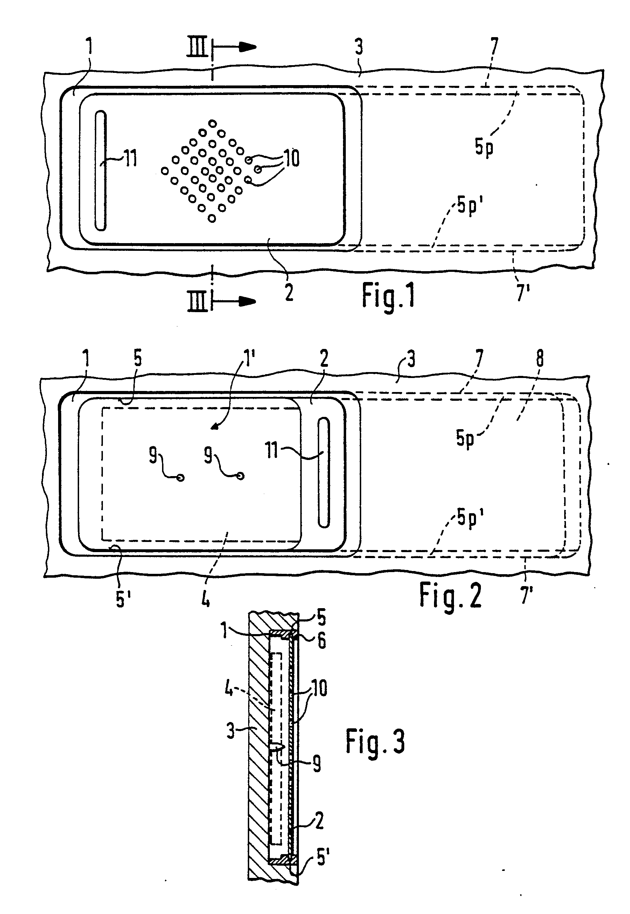

The container 1 is also provided with two projecting members 9 to mount the air freshener member 4, thereby utilizing the mechanical features of the air freshener element. Such projecting members 9, as may be seen in Figure 2, are preferably located in the centre portion of the bottom 1' of the container 1.

The cover 2, as described above, is fitted by way of the guideways 5 and 5' to the main body 1 and has an essentially rectangular shape. To facilitate the sliding movement of the cover by the user a handle 10 is provided.

The cover 2 is also provided with ventilation holes 10; both the number and the distribution of said holes 10 are designed in such a way that the diffusion of the aromatic product throughout the interior of the vehicle is enhanced.

## Claims

1. Air freshener box for an automobile, **characterised** in that said box is essentially formed by a main hollow container or body (1) incorporated in the structure of sun visor (3) of an automibile, that said container (1) is generally aligned with one of the main axes of the said sun visor (3) and is provided with a sliding cover (2) mounted in respective lateral guideways (5-5') disposed close to an upper edge (6) and extending by means of corresponding extensions sufficiently beyond the edge of the container (1) to allow for a major portion of the length of the cover (2) to be received when said cover (2) is moved into the opening position.

2. Air freshener box for an automobile according to claim 1, **characterised** by structural reinforcement means (7, 7', 8) ensuring and maintaining an appropriate solidity and rigidity of the extensions (5, 5') of the guideways (5-5') that extend beyond the perimeter of the container (1).

3. Air freshener box for an automobile according to claims 1 and 2, **characterised** in that the container (1) is provided with outwardly projecting members (9) holding the air freshener element (4).

4. Air freshener box for an automobile according to claims 1 to 3, **characterised** in that the cover (2) is provided with ventilation orifices (10) facilitating the emission of the freshener aroma and with a handle (11) so that the user can easily move the cover (2).

Fig.1

Fig. 2

Fig. 3